(19) Europäisches Patentamt
European Patent Office
Office européen des brevets

(11) **EP 2 404 372 B1**

(12) **EUROPEAN PATENT SPECIFICATION**

(45) Date of publication and mention
of the grant of the patent:
**05.05.2021 Bulletin 2021/18**

(21) Application number: **09841252.1**

(22) Date of filing: **06.03.2009**

(51) Int Cl.:
*H02M 7/217* *(2006.01)*       *H04B 1/00* *(2006.01)*
*A61N 1/02* *(2006.01)*       *A61N 1/378* *(2006.01)*
*A61N 1/36* *(2006.01)*       *H02M 7/219* *(2006.01)*

(86) International application number:
**PCT/US2009/036342**

(87) International publication number:
**WO 2010/101575 (10.09.2010 Gazette 2010/36)**

(54) **DATA AND POWER SYSTEM BASED ON CMOS BRIDGE**

DATEN- UND STROMVERSORGUNGSSYSTEM AUF DER BASIS EINER CMOS-BRÜCKE

SYSTÈME D'ALIMENTATION ET DE DONNÉES BASÉ SUR UN PONT CMOS

(84) Designated Contracting States:
**AT BE BG CH CY CZ DE DK EE ES FI FR GB GR HR HU IE IS IT LI LT LU LV MC MK MT NL NO PL PT RO SE SI SK TR**

(43) Date of publication of application:
**11.01.2012 Bulletin 2012/02**

(73) Proprietor: **MED-EL Elektromedizinische Geräte GmbH**
**6020 Innsbruck (AT)**

(72) Inventor: **ZIERHOFER, Clemens, M.**
**A-6250 Kundl (AT)**

(74) Representative: **Lucke, Andreas**
**Boehmert & Boehmert**
**Anwaltspartnerschaft mbB**
**Pettenkoferstrasse 22**
**80336 München (DE)**

(56) References cited:
**US-A- 4 355 287**       **US-A- 5 559 507**
**US-A1- 2002 003 168**       **US-A1- 2002 042 561**
**US-A1- 2007 121 355**       **US-A1- 2007 121 355**
**US-A1- 2008 177 353**

## Description

Field of the Invention

The present invention relates to a signal processing circuit, and more particularly, to a CMOS full-wave rectifier circuit that extract a data component signal.

Background Art

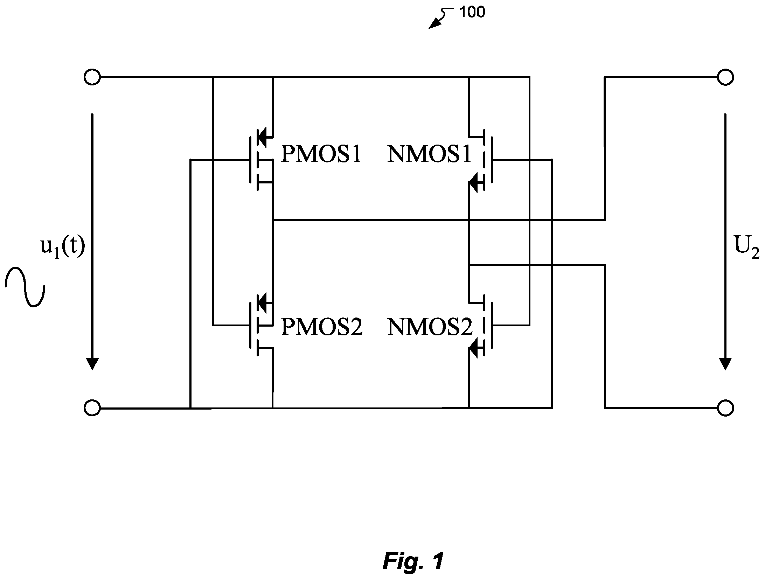

Generally, rectifiers are used for the conversion of AC to DC voltage. A CMOS bridge circuit **100** that can be used in such a rectifier is shown in Fig. 1. The CMOS bridge circuit **100** can be regarded as a non-linear, two-port device having an input voltage $u_1(t)$ that receives the AC voltage, a output voltage U2, and four CMOS switches PMOS1, PMOS2, NMOS1, and NMOS2. In general, the output port is connected to a load which may be a purely resistive load (full-wave rectifier), or a resistive load in parallel with a capacitive load (for DC voltage).

The gates of the CMOS switches may be directly connected to the input voltage terminals. Assuming a purely resistive load and an ideal switching performance of the transistors, the following conditions are fulfilled:

$$U_2 = |u_1(t)|, \quad \text{if } |u_1(t)| \geq u_{THR},$$

and

$$U_2 = 0, \quad \text{if } |u_1(t)| < u_{THR},$$

whereby voltage $u_{THR}$ denotes a MOS-threshold voltage, which here is assumed to be equal for both, PMOS and NMOS transistors. For $u_1(t) \geq u_{THR}$, PMOS1 and NMOS2 are switched on (low impedance), whereas transistor PMOS2 and NMOS1 are switched off (high impedance), and vice versa for $u_1(t) \leq -u_{THR}$, transistors PMOS2 and NMOS1 are switched on, and transistors PMOS1 and NMOS2 are switched off. Thus, for the special case of an ohmic load, the CMOS-bridge **100** of Fig. 1 represents a full-wave rectifier. Note that here the full input voltage magnitude applies at the load and there is no reduction due to diode voltage drops. Typically, MOS threshold voltages are $u_{THR} \sim 0.7V$.

Assuming a sinusoidal input voltage, the bridge circuit **100** does not fully work as a rectifier for all types of loads because transistor switches operated in ON-states allow current flow in both directions (in contrast to a diode). For example, if the circuit load is a parallel resistor and capacitor then the capacitor is partly discharged by the transistors in switch-turn-on states. Assuming $u_1(t) > u_{THR}$, PMOS1 and NMOS2 are switched on, and voltage U2 simply follows the input voltage $u_1(t)$. This means that the load capacitor is discharged not only via the load resistor, but also via the input lines. One way to address this is connect an output diode in series with the load resistor and output capacitor; however this may be unacceptable in low power applications due to the diode voltage drop.

Furthermore, when a constant DC voltage is desired, the addition of a smoothing capacitor and/or diode in conjunction with the bridge circuit may be impractical for applications that have limited space. For example, the sensing/stimulation element of a retinal implant is positioned directly within the eyeball, making space a primary concern.

A signal processing circuit according to the preamble of claim 1 is known from US 2007/0121355 A1.

US 2002/0003168 A1 discloses an integrated circuit card comprising:a contact terminal which is to be contacted with an external device and which receives a first control signal from the external device; a first control signal detection circuit which is coupled to the contact terminal, which detects the first control signal and which outputs a first detection signal; a coil which is to be magnetically coupled to the external device and which receives a second control signal from the external device; a second control signal detection circuit which is coupled to the coil, which detects the second control signal and which outputs a second detection signal; a mode direction circuit which receives the first and second detection signals and outputs a mode signal designating a contact-free mode and a contact mode, wherein the mode direction circuit outputs the mode signal designating the contact-free mode when the first detection signal is in an inactive state and the second detection signal is in an active state and wherein the mode direction circuit outputs the mode signal designating the contact mode regardless of the state of the second detection signal when the first detection signal is in the active state; and a processing circuit which receives the mode signal and which performs a predetermined operation based on the contact mode and the contact-free mode designated by the mode signal.

Summary of the Invention

The present invention provides a signal processing circuit according to claim 1 and a method for providing data and power according to claim 5. Preferred embodiments are defined in the dependent claims.

In accordance with an embodiment of the invention, a signal processing circuit includes a CMOS bridge rectifier circuit. The CMOS bridge rectifier circuit includes a first input terminal and a second input terminal for receiving a rectangular wave form that includes a data sequence. The CMOS bridge rectifier circuit further includes a first output terminal and a second output terminal for providing a rectified dc output voltage. A first data output terminal is connected to one of the first and the second input terminals, and a second data output terminal is connected to one of the first and the second output terminals, wherein the data output terminals provide an output signal representative of the data sequence.

In accordance with related embodiments of the invention, the signal processing circuit may further include a substantially resistive load (with the only output capacitance being, for example, relatively small parasitic capacitances from circuit components and leads) operatively coupled between the first and second voltage output terminals, the resistive load without a discrete parallel capacitor. The signal processing circuit may be integrated on a single chip. An implanted medical device, such as a retinal implant or a cochlear implant, may include the signal processing circuit. A chip may include the signal processing circuit, with a resistive load coupled between the first and second output terminals without a discrete parallel capacitor. The load may be a signal processor.

Also disclosed herein is a method of providing data and power in a medical implant. The method includes applying a rectangular wave input signal between a first input terminal and a second input terminal. A first switch is coupled between the first input terminal and a first node. A second switch is coupled between the second input terminal and the first node. The first node is coupled to a first output terminal. A third switch is coupled between the first input terminal and a second node. A fourth switch is coupled between the second input terminal and the second node. The second node is coupled to a second output terminal. A third output terminal is coupled to the second input terminal, and a fourth output terminal coupled to the second node. The first switch and fourth switch are gated on when the input signal is of a first polarity; and the second switch and the third switch are gated on when the input signal is of a second polarity opposite the first polarity so that the first and second output terminals provide a dc voltage, and the third and fourth terminals provide a data component.

In accordance with related embodiments of the invention, the medical implant may be a cochlear implant or a retinal implant. A substantially resistive load (with the only output capacitance being, for example, relatively small parasitic capacitances from circuit components and leads) may be operatively coupled between the first and second output terminals, the resistive load without a discrete parallel capacitor. The first switch, the second switch, the third switch, and the fourth switch may be MOS transistors. The method may further include disconnecting the input signal from the input terminals for a period of time after the switches are gated on (e.g., after applying the rectangular wave). The rectangular wave input signal may be non-periodic.

In accordance with another embodiment of the invention, a rectangular wave form that includes a data sequence is applied across a first input terminal and a second input terminal of a CMOS bridge rectifier. The CMOS bridge rectifier includes a first output terminal and a second output terminal for providing a rectified dc output voltage. A first data output terminal is connected to the first or second input terminals, and a second data output terminal is connected to the first or second output terminals. The data output terminals provide an output signal representative of the data sequence.

The medical implant may be a cochlear implant or a retinal implant. A substantially resistive load (with the only output capacitance being, for example, relatively small parasitic capacitances from circuit components and leads) may be operatively coupled between the first and second output terminals, the resistive load without a discrete parallel capacitor. The method may further include disconnecting the input signal from the input terminals for a period of time after applying the rectangular wave. The rectangular wave input signal may be non-periodic.

Further disclosed herein is a method of signal processing includes generating at a first component a rectangular wave form. The rectangular wave form is transmitted to an implanted second component via a wired interface between the first component and the second component, the second component including a CMOS bridge rectifier. The rectangular wave form is applied across a first input terminal and a second input terminal of the CMOS bridge rectifier. The CMOS bridge rectifier includes a first output terminal and a second output terminal for providing a rectified power component. A substantially resistive load (with the only output capacitance being, for example, relatively small parasitic capacitances from circuit components and leads) is operatively coupled between the first and second output terminals, the resistive load without a discrete parallel capacitor.

The second component may be implanted in the retina. The second component may include one or more electrodes, the method further including activating the one or more electrodes, wherein activating the one or more electrodes is powered, at least in part, by the rectified power component. The first component may be implanted. The first component may be implanted behind the ear.

The first and second component may be part of a cochlear implant, wherein the method includes implanting at least one of the first and second components. The second component may include a microphone powered, at least in part, by the rectified power component, the first component including an electrode array for stimulating the acoustic nerve. The rectangular wave form may include a data sequence, wherein data output terminals coupled to the CMOS bridge rectifier provide an output signal representative of the data sequence. The method may further include neural stimulation by the first and/or second components.

Also disclosed herein is a system for signal processing which includes a first component for generating and transmitting a rectangular wave form. A second component includes a CMOS bridge rectifier, the second component receiving the rectangular wave form from the first component via a wired interface between the first component and the second component. The CMOS bridge rectifier includes a first input terminal and a second input terminal for receiving the rectangular wave form. The CMOS bridge rectifier further includes a first output terminal and a second output terminal for providing a rectified power component. A substantially resistive load (with the only output capacitance being, for example, relatively small parasitic capacitances from circuit components and leads) is operatively coupled between the first and second output terminals, the resistive load without a discrete parallel capacitor.

The second component may be adapted for implantation in the retina, the second component including one or more electrodes, wherein the electrodes are activated using, at least in part, the rectified power component. In other embodiments, the first and second components may be adapted to be part of a cochlear implant, the second component including a microphone powered, at least in part, by the rectified power component, the first component including an electrode array for stimulating the acoustic nerve. The rectangular wave form may include a data sequence, wherein data output terminals coupled to the CMOS bridge rectifier provide an output signal representative of the data sequence. The first component may include a first housing, with the second component includes a second housing different from the first housing.

Brief Description of the Drawings

Fig. 1 is a schematic showing a CMOS bridge circuit with sinusoidal input (Prior Art);

Fig. 2 is a schematic showing a CMOS bridge circuit with a rectangular wave input, in accordance with one embodiment of the invention;

Fig. 3 shows a rectangular wave input signal having active and floating periods, in accordance with one embodiment of the invention;

Fig. 4 is a schematic showing a power supply system for a retinal implant, in accordance with one embodiment of the invention;

Fig. 5 shows a pulsed input signal that may be used in the embodiment shown in Fig. 4, in accordance with one embodiment of the invention;

Fig. 6 is a schematic showing a CMOS bridge circuit driven providing both a power component and a data component, in accordance to one embodiment of the invention;

Fig. 7 is a schematic showing a bridge circuit implemented by two inverters that provides both a power component and a data component and

Figures 8A and 8B are schematics showing a transmission system.

Detailed Description of Specific Embodiments

Operation of a bridge circuit as described above is typically considered with respect to a sine wave ac input signal such as 110 vac 60 Hz used for appliances in the United States. But the behavior of the circuit is interestingly different when the input signal is some form of a rectangular wave signal. The rectangular wave may be, for example, periodic, and/or a non-periodic signal that may include a data component (i.e., carrying information).

For example, in the general case of a rectangular wave input signal, a CMOS bridge circuit may be used to provide a constant DC voltage without necessarily requiring a discrete smoothing output capacitor and/or additional diodes. The load may be substantially resistive; with the only output capacitance being, for example, relatively small parasitic capacitances from circuit components and leads. Applying a rectangular wave input signal to a CMOS bridge circuit without the need for a discrete smoothing capacitor or diodes may advantageously be used in applications with limited space and weight.

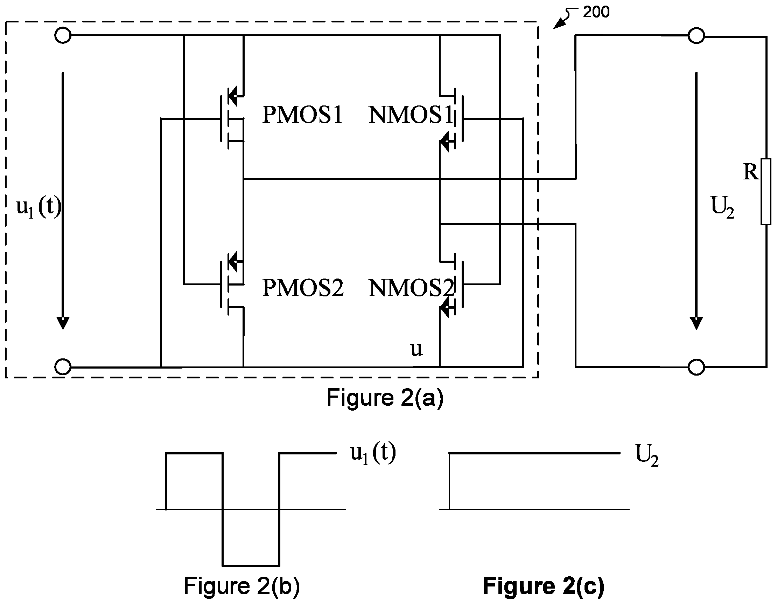

Fig. 2(a) is a schematic showing a CMOS bridge circuit **200** with a rectangular wave input $u_1(t)$ and no output capacitor, in accordance with one embodiment of the invention. The CMOS bridge circuit **200** includes an output voltage U2, and four CMOS switches PMOS1, PMOS2, NMOS1, and NMOS2. The output port is connected to a load **R** which is substantially resistive. Assuming $u_1(t)$ is equal to $\pm$ X volts $\geq u_{THR}$, as shown in Fig. 2(b), then $U_2 = |u_1(t)| = $ X volts, a constant DC output voltage U2, as shown in Fig. 2(c).

In illustrative embodiments of the invention, the CMOS bridge circuit may be used, without limitation, in a retinal implant. The central component of the retinal implant is typically an electronic chip located either on the surface of the inner retina (epiretinal approach), or in the subretinal space (subretinal approach). Typically, the size of this chip is some square millimeters, and the thickness is some tens of microns. There is minimal room for additional electrical components, and the entire functionality of the retinal implant has to be integrated on chip. Therefore, for a retinal implant, not having a discrete capacitor is critical.

The chip of the retinal implant may essentially have an array of subunits, where each subunit consists of a photodiode, an analog amplifier and a stimulating electrode. These subunits are designed to convert the light energy from images into electrical impulses to stimulate the remaining functional cells of the retina. Unfortunately, early hopes that such a chip could be powered solely by incident light and not require the use of external power supply did not hold.

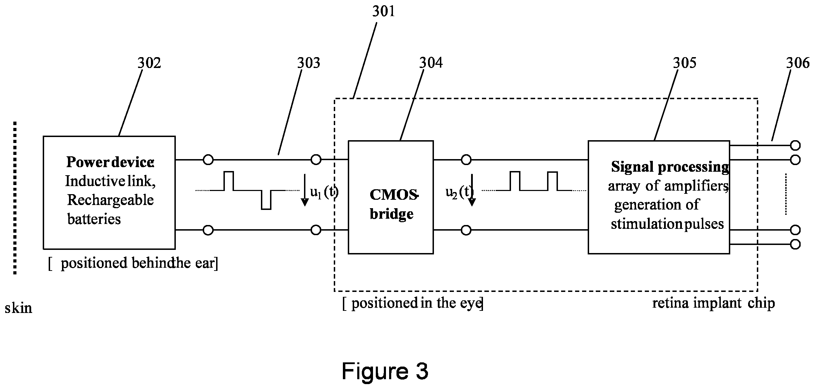

Fig.3 shows a retinal chip **301** connected to a second device **302** providing power- and control signals, in accordance with one embodiment of the invention. The connection between the retinal chip **301** and the second device is established by wire (unlike Rf-based transmission systems that deal with sinusoidal waves). For example, the second device **302** may be implanted in the area behind the ear (similar to a cochlear implant), and include rechargeable batteries, which in turn can be recharged - if required - transcutaneously using an inductive link. The electrode array **306** provides stimulation patterns to elicit optical impressions in the brain. Wires **303** connecting the retinal chip **301** and device **302** may have a length of some centimeters.

A direct supply of the retinal chip with dc-voltage should be avoided, because wires **303** with dc-voltage potentials in aggressive body fluids are problematic for a variety of reasons. For example, although the wires are isolated against each other, a permanent electrical field between wires may cause material migration and lead to the growth of low impedance bridges (dentrites). Another problem might occur, if the isolation between two wires is defect for some reason. Then the dc-potential between wires might cause electrolysis and possibly can destroy nerve tissue.

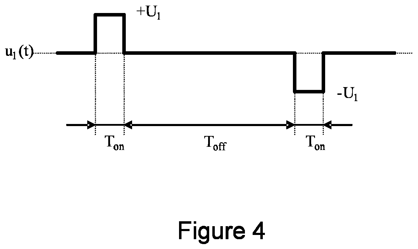

DC-potentials in the power supply wires **303** are avoided, if device **302** generates pulsed waveforms as shown in Fig.4. In Fig. 4, voltage $u_1(t)$ is composed of segments with constant positive and negative levels $+U_1$ and $-U_1$ of durations $T_{on}$, respectively, and voltage $u_1(t)$ = 0 for periods $T_{off}$. It is assumed that the retinal chip **301** is activated during $T_{on}$, and deactivated during $T_{off}$. Typically, the activation rate could be about 20Hz, according to the requirements of the human optical systems. For such a pulsed input voltage, the CMOS-bridge **304** generates a rectified version of the input voltage, i.e., the output voltage is $u_2(t) \sim +U_1$ during $T_{on}$, and $u_2(t)$ = 0 during $T_{off}$. Thus $u_2(t)$ can serve as a pulsed supply voltage for the signal processing portion of retinal chip **301**. Alternatively, if a continuous rectangular wave is input, $u_2(t)$ can serve as a continuous DC supply voltage.

Both the CMOS bridge circuit **304** and its load may advantageously be integrated on single chip. For example, the bridge circuit **304** may be functionally coupled with other circuitry such as a signal processor **305**, and both the bridge circuit **304** and the signal processing circuitry **305** can be integrated onto a single chip.

The CMOS bridge circuit may also be used, without limitation, in a microphone subsystem within a cochlear implant. For example, in a totally implantable cochlear implant, the microphone typically is positioned outside the main device, with power provided by the main device to the microphone via two wires. As with the retinal implant, size and weight constraints apply and there is minimal space for any additional circuit components. A direct supply of DC voltage to the microphone is potentially dangerous to surrounding tissue, as described above with reference to the retinal implant. A rectangular wave input in combination with the CMOS bridge circuit is therefore ideal.

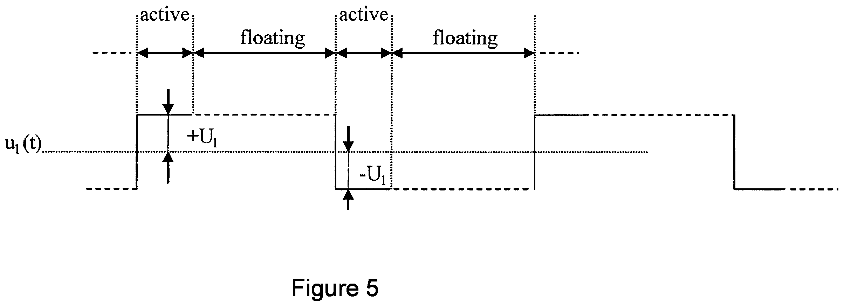

When the input terminals have a high impedance across them (e.g., as in the case where they are unconnected), the CMOS bridge circuit also possesses the interesting property of remaining stable in its existing logic state. For example, as shown in Fig. 5, assume that a +5 vdc input is applied to the input terminals of the bridge circuit in Fig. 1 during the time period on the left of Fig. 2 labeled as "active." Then, the same +5 vdc will be passed to the output terminals across the load resistor and optionally, any output capacitor. Assuming that the input signal is then disconnected from the input terminals, PMOS1 and NMOS2 will remain in a low impedance state (and assuming an RC time constant of the load resistor and any output capacitor are sufficiently large) the output voltage will continue to float at +5 vdc due to the output capacitor. A similar action occurs oppositely on the right side of Fig. 5 during the second active and floating periods. This may exploited in some situations such as low power applications when it may be possible to apply the input signal for relatively short active periods and let the circuit float during succeeding inactive periods. Such a signal having active and floating periods need not necessarily be periodic, but in some applications may be non-periodic signal such as an input signal having a data component.

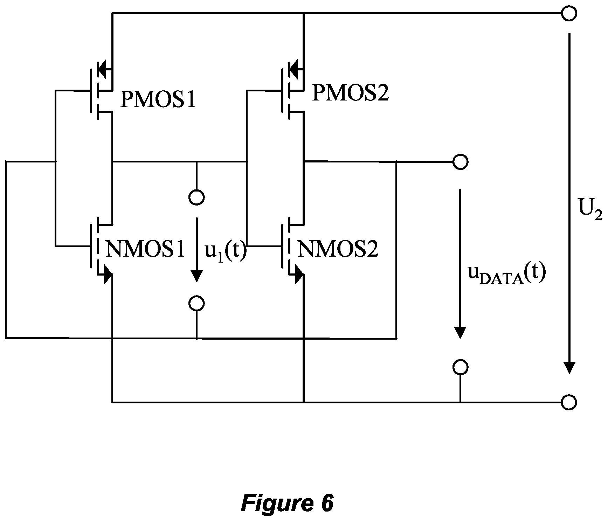

Further illustrative embodiments of the present invention drive a CMOS bridge circuit with a rectangular input signal so as to provide both a power component and a data component, in accordance to one embodiment of the invention. For example, as shown in Fig. 6, a continuous rectangular driving signal $u_1(t)$ may be applied to the CMOS bridge circuit to provide a constant power component U2, similar to above described embodiments. In addition, a rectangular signal $u_{DATA}(t)$ output is coupled, without limitation, to one of the input terminals of the CMOS bridge circuit, and one of the U2 output terminals of the CMOS bridge circuit, to derive a data component. Note that both U2 and $u_{DATA}(t)$ are referenced to the same common reference potential.

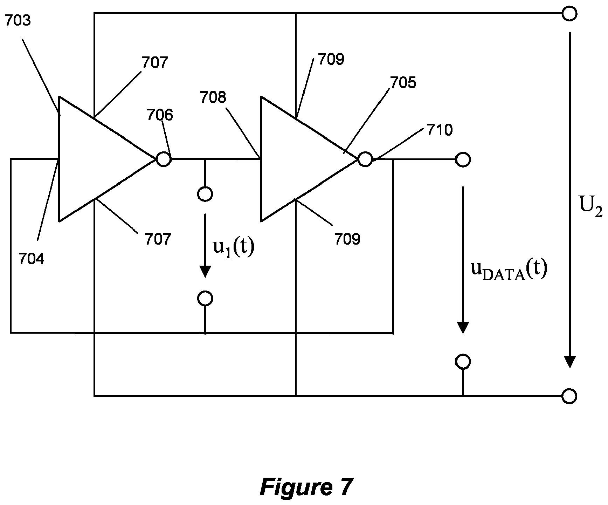

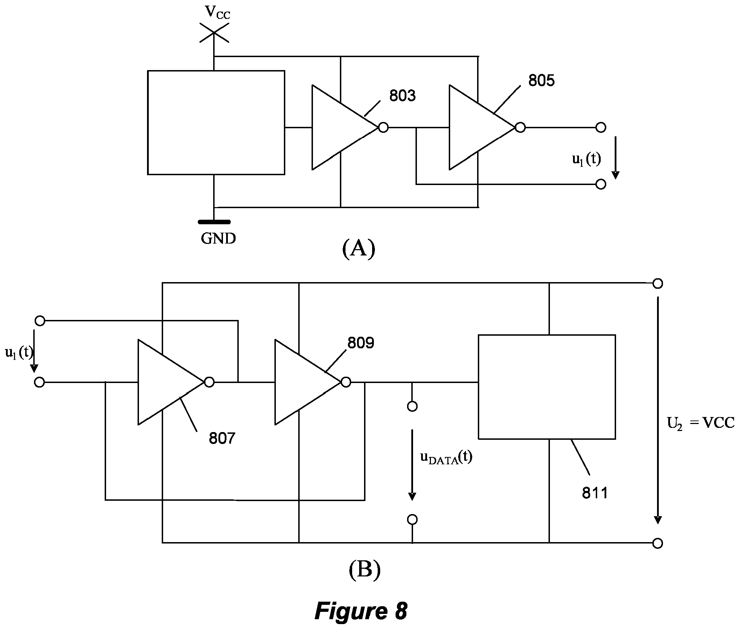

The following examples described in respect to Fig. 7 and 8 are not according to the invention and are present for illustration purposes only. Fig. 7 is similar to the circuit shown in Fig. 6, implemented with two inverter circuits. More particuarly, the signal processing circuit includes an input inverter **703** and an output inverter **705**. Each inverter **703** and **705** includes a signal input **704** and **708,** respectively, for receiving an input (e.g., a rectangular wave), and a signal output **706** and **710,** respectively, for providing an output (e.g., an inverted rectangular wave). Each inverter **703** and **705** also includes a pair of voltage outputs **707** and **709** for developing a rectified DC output voltage. A first circuit input terminal is connected to the output **706** of the input inverter **703** and the input **708** of the output inverter **705**. A second circuit input terminal is connected to the input **704** of the input inverter **703** and the output **710** of the output inverter **705,** wherein the signal input terminals receive an input signal having a data component. A pair of supply voltage output terminals U2 are connected to the voltage output terminals **707** and **709** of the inverters **703** and **705** for providing a rectified dc supply voltage output. A first circuit output terminal is connected to one of the supply voltage output terminals. A second circuit output terminal is connected to the second circuit input terminal. The circuit output terminals $u_{DATA}(t)$ provide an output signal including the data component.

Figs. 8A and 8B are schematics showing a transmission system. The primary system shown in Fig. 8(a) includes a signal generator **801** that generates the rectangular signal $u_1(t)$ from a data sequence by using, without limitation, two inverters **803** and **805**. The rectangular system transmits the rectangular signal $u_1(t)$ to a secondary system, shown in Fig. 8(b). In various embodiments, the rectangular signal $u_1(t)$ is transmitted, without limitation, via a wired interface between the primary system and the secondary system.

The secondary system includes a CMOS bridge rectifier for deriving the power supply voltage U2 and the data signal $u_{DATA}(t)$. The CMOS bridge rectifier may use, without limitation, two inverters **807** and **809**, similar to the embodiment of Fig. 7. The data signal $u_{DATA}(t)$ may be used for further signal detection and processing **811**. The CMOS bridge rectifier advantageously may be coupled to a substantially resistive load that does not include a discrete parallel capacitor. As a special feature of the system: if $u_1(t)$ changes from a particular defined state to a floating state, e.g., by disconnecting the lines of $u_1(t)$ from any defined potential, then $u_{DATA}(t)$ remains in a stable the state, as described above in connection with Fig. 5.

The first and secondary systems shown in Figs. 8A and 8B may be used to form a multi-component implant that is particularly advantageous when a particular component of the implant has limited space. For example, as described above the first component of a retinal implant may act as a primary system and provide rectangular signal $u_1(t)$. The first component may be located, without limitation, behind the ear, so that power can be transmitted transcutaneously using an inductive link. The second component of the retinal implant may then act as the secondary system that receives the rectangular signal $u_1(t)$ and uses a CMOS bridge rectifier to derive power and activate one or more electrodes. In other embodiments, the first and second components may be re adapted to be part of a cochlear implant, the second component including a microphone powered, at least in part, by the rectified power component, the first component including an electrode array for stimulating the acoustic nerve.

The signal processing circuit in the above-described embodiments may advantageously be used in a wide variety of applications. For example, it may be used to provide a desired supply voltage polarity and data component signals in diverse fields such as, without limitation, the automotive or medical fields. Embodiments may also include using such a circuit as the basis for a polarity protection data circuit which allows for arbitrary connecting of the inputs to a dc source, independently of the polarity.

In various embodiments, the disclosed method may be implemented as a computer program product for use with a computer system. Such implementation may include a series of computer instructions fixed either on a tangible medium, such as a computer readable media (e.g., a diskette, CD-ROM, ROM, or fixed disk) or transmittable to a computer system, via a modem or other interface device, such as a communications adapter connected to a network over a medium. Medium may be either a tangible medium (e.g., optical or analog communications lines) or a medium implemented with wireless techniques (e.g., microwave, infrared or other transmission techniques). The series of computer instructions embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable media with accompanying printed or electronic documentation (e.g., shrink wrapped software), preloaded with a computer system (e.g., on system ROM or fixed disk), or distributed from a server or electronic bulletin board over the network (e.g., the Internet or World Wide Web).

**Claims**

1.  A signal processing circuit comprising:
    a CMOS bridge rectifier circuit including:

        a first input terminal and a second input terminal for receiving a rectangular wave form that includes a data sequence;
        a first output terminal and a second output terminal for providing a rectified dc output voltage;
        wherein a first switch is coupled between the first input terminal and a first node, a second switch is coupled between the second input terminal and the first node, the first node is coupled to said first output terminal, a third switch is coupled between the first input terminal and a second node, a fourth switch is coupled between the second input terminal and the second node; the second node is coupled to said second output terminal;
        wherein the first switch and fourth switch are gated on when the input signal is of a first polarity; and wherein the second switch and the third switch are gated on when the input signal is of a second polarity opposite the first polarity so that the first and second output terminals provide the dc voltage, and **characterized by**
a first data output terminal connected to the second input terminal, and a second data output terminal connected to the second output terminal, wherein the data output terminals provide an output signal representative of the data sequence.

2. The signal processing circuit according to claim 1, further comprising a substantially resistive load operatively coupled between the first and second voltage output terminals, the resistive load without a discrete parallel capacitor, wherein the signal processing circuit is preferably integrated on a single chip.

3. A medical device comprising the signal processing circuit of claim 1, wherein the medical device is in particular one of a retinal implant and a cochlear implant.

4. A chip comprising:

the signal processing circuit according to claim 1; and
a substantially resistive load coupled between the first and second output terminals without a discrete parallel capacitor, wherein the load preferably includes a signal processor.

5. A method of providing data and power, the method comprising:

applying a rectangular wave form that includes a data sequence across a first input terminal and a second input terminal of a CMOS bridge rectifier, the CMOS bridge rectifier including a first output terminal and a second output terminal for providing a rectified dc output voltage,
wherein a first switch is coupled between the first input terminal and a first node, a second switch is coupled between the second input terminal and the first node, the first node coupled is to said first output terminal, a third switch is coupled between the first input terminal and a second node, a fourth switch is coupled between the second input terminal and the second node; the second node is coupled to said second output terminal;
wherein the first switch and fourth switch are gated on when the input signal is of a first polarity; and wherein the second switch and the third switch are gated on when the input signal is of a second polarity opposite the first polarity so that the first and second output terminals provide the dc voltage, **characterized by** a first data output terminal connected to the second input terminal, and a second data output terminal connected to the second output terminal, the data output terminals providing an output signal representative of the data sequence.

6. The method of claim 5, wherein a substantially resistive load is operatively coupled between the first and second output terminals, the resistive load without a discrete parallel capacitor.

7. The method of claim 5 or 6, wherein the method further comprises:
disconnecting the input signal from the input terminals for a period of time after applying the rectangular wave, and/or wherein the rectangular wave input signal is non-periodic.

8. The method of claim 5, the method comprising:

generating at a first component said rectangular wave form;
transmitting the rectangular wave form to an implanted second component via a wired interface between the first component and the second component, the second component including said CMOS bridge rectifier; wherein a substantially resistive load is operatively coupled between the first and second output terminals, the resistive load without a discrete parallel capacitor.

9. The method according to claim 8, wherein the second component includes one or more electrodes, the method further comprising activating the one or more electrodes, and wherein activating the one or more electrodes is powered, at least in part, by the rectified power component.

10. A system for signal processing, the system comprising the signal processing circuit of claim 1 and:

a first component for generating and transmitting said rectangular wave form;
a second component including said CMOS bridge rectifier, the second component receiving the rectangular wave form from the first component via a wired interface between the first component and the second component, wherein a substantially resistive load is operatively coupled between the first and second output terminals, the resistive load without a discrete parallel capacitor.

11. The system according to claim 10, wherein the second component includes one or more electrodes, wherein the electrodes are activated using, at least in part, the rectified power component.

**Patentansprüche**

1. Signalverarbeitungsschaltung, die folgendes umfasst:

   eine CMOS-Brückengleichrichterschaltung, umfassend:

   einem ersten Eingangsanschluss und einem zweiten Eingangsanschluss zum Empfangen einer rechteckigen Wellenform, die eine Datensequenz enthält;
   einen ersten Ausgangsanschluss und einen zweiten Ausgangsanschluss zum Bereitstellen einer gleichgerichteten Ausgangs-Gleichspannung;
   wobei ein erster Schalter zwischen dem ersten Eingangsanschluss und einem ersten Knotenpunkt gekoppelt ist, ein zweiter Schalter zwischen dem zweiten Eingangsanschluss und dem ersten Knotenpunkt gekoppelt ist, der erste Knotenpunkt mit dem ersten Ausgangsanschluss gekoppelt ist, ein dritter Schalter zwischen dem ersten Eingangsanschluss und einem zweiten Knotenpunkt gekoppelt ist, ein vierter Schalter zwischen dem zweiten Eingangsanschluss und dem zweiten Knotenpunkt gekoppelt ist; der zweite Knotenpunkt mit dem zweiten Ausgangsanschluss gekoppelt ist; wobei der erste Schalter und der vierte Schalter durchgeschaltet sind, wenn das Eingangssignal eine erste Polarität aufweist; und wobei der zweite Schalter und der dritte Schalter durchgeschaltet sind, wenn das Eingangssignal eine zweite, der ersten Polarität entgegengesetzte Polarität aufweist, so dass der erste und der zweite Ausgangsanschluss die Gleichspannung bereitstellen, und **gekennzeichnet durch** einen ersten Datenausgangsanschluss, der mit dem zweiten Eingangsanschluss verbunden ist, und einen zweiten Datenausgangsanschluss, der mit dem zweiten Ausgangsanschluss verbunden ist, wobei die Datenausgangsanschlüsse ein Ausgangssignal bereitstellen, das die Datenfolge repräsentiert.

2. Signalverarbeitungsschaltung nach Anspruch 1, ferner umfassend eine im Wesentlichen resistive Last, die operativ zwischen den ersten und zweiten Spannungsausgangsanschluss gekoppelt ist, wobei die resistive Last keinen diskreten Parallelkondensator aufweist, wobei die Signalverarbeitungsschaltung vorzugsweise auf einem einzigen Chip integriert ist.

3. Medizinisches Gerät umfassend die Signalverarbeitungsschaltung nach Anspruch 1, wobei das medizinische Gerät insbesondere ein Netzhautimplantat oder ein Cochlea-Implantat ist.

4. Chip umfassend:

   die signalverarbeitende Schaltung nach Anspruch 1; und
   eine im Wesentlichen ohmsche Last, die zwischen dem ersten und dem zweiten Ausgangsanschluss ohne einen diskreten parallelen Kondensator gekoppelt ist, wobei die Last vorzugsweise einen Signalprozessor umfasst.

5. Verfahren zum Bereitstellen von Daten und Leistung, wobei das Verfahren folgendes umfasst:

   Anlegen einer rechteckigen Wellenform, die eine Datensequenz umfasst, zwischen einen ersten Eingangsanschluss und einen zweiten Eingangsanschluss eines CMOS-Brückengleichrichters, wobei der CMOS-Brückengleichrichter einen ersten Ausgangsanschluss und einen zweiten Ausgangsanschluss zum Bereitstellen einer gleichgerichteten Ausgangs-Gleichspannung umfasst,
   wobei ein erster Schalter zwischen dem ersten Eingangsanschluss und einem ersten Knotenpunkt gekoppelt ist, ein zweiter Schalter zwischen dem zweiten Eingangsanschluss und dem ersten Knotenpunkt gekoppelt ist, der erste Knotenpunkt mit dem ersten Ausgangsanschluss gekoppelt ist, ein dritter Schalter zwischen dem ersten Eingangsanschluss und einem zweiten Knotenpunkt gekoppelt ist, ein vierter Schalter zwischen dem zweiten Eingangsanschluss und dem zweiten Knotenpunkt gekoppelt ist; der zweite Knotenpunkt mit dem zweiten Ausgangsanschluss gekoppelt ist; wobei der erste Schalter und der vierte Schalter durchgeschaltet sind, wenn das Eingangssignal eine erste Polarität aufweist; und wobei der zweite Schalter und der dritte Schalter durchgeschaltet sind, wenn das Eingangssignal eine zweite Polarität aufweist, die der ersten Polarität entgegengesetzt ist, so dass der erste und der zweite Ausgangsanschluss die Gleichspannung bereitstellen, **gekennzeichnet durch** einen ersten Datenausgangsanschluss, der mit dem zweiten Eingangsanschluss verbunden ist, und einen zweiten Datenausgangsanschluss, der mit dem zweiten Ausgangsanschluss verbunden ist, wobei die Datenausgangsanschlüsse ein Ausgangssignal bereitstellen, das die Datensequenz repräsentiert.

6. Verfahren nach Anspruch 5, wobei eine im Wesentlichen ohmsche Last operativ zwischen den ersten und zweiten Ausgangsanschluss gekoppelt ist, wobei die ohmsche Last keinen diskreten parallelen Kondensator aufweist.

**7.** Verfahren nach Anspruch 5 oder 6, wobei das Verfahren ferner folgendes umfasst:
Trennen des Eingangssignals von den Eingangsanschlüssen für eine Zeitspanne nach Anlegen der Rechteckwelle, und/oder wobei das Rechteckwellen-Eingangssignal nicht-periodisch ist.

**8.** Verfahren nach Anspruch 5, wobei das Verfahren folgendes umfasst:

Erzeugen der rechteckigen Wellenform an einer ersten Komponente;
Übertragen der rechteckigen Wellenform an ein implantiertes zweites Bauteil über eine verdrahtete Schnittstelle zwischen dem ersten Bauteil und dem zweiten Bauteil, wobei das zweite Bauteil den CMOS-Brückengleichrichter umfasst; wobei eine im Wesentlichen ohmsche Last operativ zwischen den ersten und den zweiten Ausgangsanschluss gekoppelt ist, wobei die ohmsche Last keinen diskreten parallelen Kondensator umfasst.

**9.** Verfahren nach Anspruch 8, wobei die zweite Komponente eine oder mehrere Elektroden umfasst, wobei das Verfahren ferner das Aktivieren der einen oder mehreren Elektroden umfasst, und wobei das Aktivieren der einen oder mehreren Elektroden zumindest teilweise durch die gleichgerichtete Leistungskomponente betrieben wird.

**10.** System zur Signalverarbeitung, wobei das System die Signalverarbeitungsschaltung nach Anspruch 1 umfasst und:

eine erste Komponente zum Erzeugen und Übertragen der rechteckigen Wellenform;
eine zweite Komponente, die den CMOS-Brückengleichrichter umfasst, wobei die zweite Komponente die rechteckige Wellenform von der ersten Komponente über eine verdrahtete Schnittstelle zwischen der ersten Komponente und der zweiten Komponente empfängt, wobei eine im Wesentlichen ohmsche Last operativ zwischen den ersten und den zweiten Ausgangsanschluss gekoppelt ist, wobei die ohmsche Last keinen diskreten parallelen Kondensator aufweist.

**11.** System nach Anspruch 10, wobei die zweite Komponente eine oder mehrere Elektroden umfasst, wobei die Elektroden zumindest teilweise unter Verwendung der gleichgerichteten Leistungskomponente aktiviert werden.

**Revendications**

**1.** Circuit de traitement de signal comprenant :
un circuit redresseur en pont semi-conducteur à oxyde de métal complémentaire, CMOS, comprenant :

une première borne d'entrée et une seconde borne d'entrée destinées à recevoir une forme d'onde rectangulaire qui inclut une séquence de données ;
une première borne de sortie et une seconde borne de sortie destinées à fournir une tension de sortie continue redressée ;
dans lequel un premier commutateur est couplé entre la première borne d'entrée et un premier nœud, un deuxième commutateur est couplé entre la seconde borne d'entrée et le premier nœud, le premier nœud est couplé à ladite première borne de sortie, un troisième commutateur est couplé entre la première borne d'entrée et un second nœud, un quatrième commutateur est couplé entre la seconde borne d'entrée et le second nœud, et le second nœud est couplé à ladite seconde borne de sortie ;
dans lequel le premier commutateur et le quatrième commutateur sont déclenchés périodiquement lorsque le signal d'entrée est d'une première polarité ; et dans lequel le deuxième commutateur et le troisième commutateur sont déclenchés périodiquement lorsque le signal d'entrée est d'une seconde polarité opposée à la première polarité, de sorte que les première et seconde bornes de sortie fournissent la tension continue, et **caractérisé par** :
une première borne de sortie de données connectée à la seconde borne d'entrée, et une seconde borne de sortie de données connectée à la seconde borne de sortie, dans lequel les bornes de sortie de données fournissent un signal de sortie représentatif de la séquence de données.

**2.** Circuit de traitement de signal selon la revendication 1, comprenant en outre une charge sensiblement résistive couplée fonctionnellement entre les première et seconde bornes de sortie de tension, la charge résistive étant démunie de condensateur parallèle discret, dans lequel le circuit de traitement de signal est de préférence intégré sur une unique puce.

**3.** Dispositif médical comprenant le circuit de traitement de signal selon la revendication 1, dans lequel le dispositif médical correspond en particulier à l'un parmi implant rétinien ou un implant cochléaire.

**4.** Puce comprenant :

le circuit de traitement de signal selon la revendication 1 ; et

une charge sensiblement résistive, couplée entre les première et seconde bornes de sortie, démunie de condensateur parallèle discret, dans lequel la charge inclut de préférence un processeur de signal.

**5.** Procédé de fourniture de données et d'énergie, le procédé comprenant l'étape ci-dessous consistant à :

appliquer une forme d'onde rectangulaire qui inclut une séquence de données, à travers une première borne d'entrée et une seconde borne d'entrée d'un redresseur en pont CMOS, le redresseur en pont CMOS incluant une première borne de sortie et une seconde borne de sortie destinées à fournir une tension de sortie continue redressée ;

dans lequel un premier commutateur est couplé entre la première borne d'entrée et un premier nœud, un deuxième commutateur est couplé entre la seconde borne d'entrée et le premier nœud, le premier nœud est couplé à ladite première borne de sortie, un troisième commutateur est couplé entre la première borne d'entrée et un second nœud, un quatrième commutateur est couplé entre la seconde borne d'entrée et le second nœud ; et le second nœud est couplé à ladite seconde borne de sortie ;

dans lequel le premier commutateur et le quatrième commutateur sont déclenchés périodiquement lorsque le signal d'entrée est d'une première polarité ; et dans lequel le deuxième commutateur et le troisième commutateur sont déclenchés périodiquement lorsque le signal d'entrée est d'une seconde polarité opposée à la première polarité, de sorte que les première et seconde bornes de sortie fournissent la tension continue, **caractérisé par** une première borne de sortie de données connectée à la seconde borne d'entrée, et une seconde borne de sortie de données connectée à la seconde borne de sortie, les bornes de sortie de données fournissant un signal de sortie représentatif de la séquence de données.

**6.** Procédé selon la revendication 5, dans lequel une charge sensiblement résistive est couplée fonctionnellement entre les première et seconde bornes de sortie, la charge résistive étant démunie de condensateur parallèle discret.

**7.** Procédé selon la revendication 5 ou 6, dans lequel le procédé comprend en outre l'étape ci-dessous consistant à :

déconnecter le signal d'entrée des bornes d'entrée, pendant une période de temps après l'application de l'onde rectangulaire, et/ou dans lequel le signal d'entrée d'onde rectangulaire est non périodique.

**8.** Procédé selon la revendication 5, le procédé comprenant les étapes ci-dessous consistant à :

générer, au niveau d'un premier composant, ladite forme d'onde rectangulaire ;

transmettre la forme d'onde rectangulaire à un second composant implanté, par l'intermédiaire d'une interface câblée entre le premier composant et le second composant, le second composant incluant ledit redresseur en pont CMOS ;

dans lequel une charge sensiblement résistive est couplée fonctionnellement entre les première et seconde bornes de sortie, la charge résistive étant démunie de condensateur parallèle discret.

**9.** Procédé selon la revendication 8, dans lequel le second composant inclut une ou plusieurs électrodes, le procédé comprenant en outre l'étape consistant à activer ladite une ou lesdites plusieurs électrodes, et dans lequel l'activation de ladite une ou desdites plusieurs électrodes est alimentée, au moins en partie, par le composant à courant redressé.

**10.** Système de traitement de signal, le système comprenant le circuit de traitement de signal selon la revendication 1, et comprenant :

un premier composant destiné à générer et transmettre ladite forme d'onde rectangulaire ;

un second composant incluant ledit redresseur en pont CMOS, le second composant recevant la forme d'onde rectangulaire en provenance du premier composant, par l'intermédiaire d'une interface câblée entre le premier composant et le second composant, dans lequel une charge sensiblement résistive est couplée fonctionnellement entre les première et seconde bornes de sortie, la charge résistive étant démunie de condensateur parallèle discret.

**11.** Système selon la revendication 10, dans lequel le second composant inclut une ou plusieurs électrodes, dans lequel les électrodes sont activées en utilisant, au moins en partie, le composant à courant redressé.

Fig. 1

R

$U_2$

200

PMOS1 NMOS1

PMOS2 NMOS2

u

$u_1(t)$

Figure 2(a)

$U_2$

Figure 2(c)

$u_1(t)$

Figure 2(b)

**Figure 3**

306

305

Signal processing
array of amplifiers
generation of
stimulation pulses

retina implant chip

$u_2(t)$

304

CMOS
bridge

301

[ positioned in the eye]

303

$u_1(t)$

302

Power device
Inductive link,
Rechargeable
batteries

[ positioned behind the ear]

skin

Figure 4

Figure 5

EP 2 404 372 B1

*Figure 6*

EP 2 404 372 B1

$U_2$

$u_{DATA}(t)$

710

705

709

709

708

$u_1(t)$

706

707

707

703

704

**Figure 7**

Figure 8

**EP 2 404 372 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070121355 A1

- US 20020003168 A1